# EUROPEAN PATENT APPLICATION

(11) **EP 3 064 210 A1**
(43) Date of publication of application: **07.09.2016**
(21) Application number: 13896715.3
(22) Date of filing: 08.11.2013
(51) Int. Cl.: A61K 31/7048, A61K 31/164, A61K 36/82, A61K 47/14, A61K 9/02, A61P 29/00

(54) **AGENT IN SUPPOSITORY FORM FOR THE TREATMENT OF HAEMORRHOIDS, PROCTITIS AND OTHER INFLAMMATORY PROCTOLOGICAL DISORDERS**

(30) Priority: 30.10.2013 RU 2013148388
(71) Applicant: Obschestvo S Ogranichennoi Otvetstvennostyu "Gemodan", Moscow 115184 (RU)
(72) Inventor: FROLOV, Dmitry Viktorovich, Moscow 117393 (RU)
(74) Representative: Bucher, Ralf Christian
(86) International application number: PCT/RU2013/000993
(87) International publication number: WO 2015/065231

(57) **Abstract**

The invention relates to an agent for the treatment of haemorrhoids, proctitis and other inflammatory proctological disorders. Said agent is produced in the form of a suppository and contains diosmin in an amount of 0.3-0.65 g, dexpanthenol in an amount of 0.0135-0.1825 g, and fatty acid glycerides. The claimed invention provides a complex anti-inflammatory, antimicrobial, local anaesthetic, adaptogenic and regenerative effect, and also exhibits good permeative ability.

## Description

### Technical field of the invention

The present invention relates to chemo pharmaceutical industry and represents a medicinal agent made as rectal suppositories to treat hemorrhoid, proctitis and other inflammatory proctology diseases. The medicinal agent relates to preparations that control veinlet tonicity, improve local trophicity of tissues and reduce inflammatory reactions in tissues.

The invention provides for anti-inflammatory, antimicrobial, local anesthetic effects, as well as a good penetrating power.

### Background of the invention

The multiplicity of medicinal preparations used in the treatment of hemorrhoid, proctitis and other proctology diseases attests that the treatment of the last has remained until now an unsolved problem of modem medicine. To treat hemorrhoid, proctitis and other proctology diseases, many medicinal preparations are used including preparations of vegetal origin. An important place in a combined treatment is reserved to efficient and, what is the most important, to safe suppositories with natural ingredients that show a wide range of biological activity thanks to a complex biological composition. Natural ingredients are widely used in official and folk medicine to prevent and to treat various diseases.

As prior art, the patent FR 6967M, 1969, is known which for the first time discloses the possibility to use diosmin (7-[[6-O-(-desoxy-alpha-L-mannopyranosyl)-beta-D-glucopyranosyl]oxy]-5-hydroxy-2-(3-hydroxy-4-methoxyphenyl)-4H-1-benzopyran-4-one), obtained by dehydrogenation of hesperidin (3',5,7-trihydroxy-4'-methoxyflavonone-7-rhamnoglucoside), as well as a pharmaceutical composition comprising diosmin as an active ingredient used alone and in combination with hesperidin and with citroflavonoids to treat pathologic conditions associated with capillary fragility. This known pharmaceutical composition can be made under various medicinal presentations: capsules, tablets, granules, suspensions, creams, suppositories. The diosmin : hesperidin : citroflavonoids ratio of the composition is 1:1:0.5 (in mass parts).

Tablets of the known invention:

| | |
|---|---|
| Diosmin | 100 mg |
| Hesperidin | 100 mg |
| Citroflavonoids | 50 mg |
| Rice starch | 40 mg |
| Talc | Balance. |

Suppositories of the known invention comprise:

| | |
|---|---|
| Diosmin | 0.100 g |
| Hesperidin | 0.050 g |
| Citroflavonoids | 0.050 g |
| Semi synthetic glycerides to 3 g. | |

The pharmacologic effect of diosmin is manifested by an increase in venous tonicity, capillary resistance, by reduced venous congestion and capillary permeability as well as by an improved microcirculation (Register of medicines of Russia. Encyclopedia of medicines. Moscow: RLS-2002, p. 305).

Diosmin-bioflavonoid combined with hesperidin provide for vein toning up and angioprotective effects, increase the tonicity and reduce the vein elasticity, which favors a lower venous congestion, decreases the permeability of capillaries and increases their resistance, together with the improved microcirculation and lymph drainage. The combination of diosmin with hesperidin provides for a rather high pharmacological effect. Nevertheless, the use of hesperidin, according to the notice, can cause digestion disorders manifested as eructation, heartburn and a feeling of pressure in the stomach, as well as symptoms of neurovegetative disorders. The use of preparations containing hersperidin, in cases of hemorrhoid exacerbation, does not supplant a specific treatment of other anal disorders.

Prior art cites the patent for an invention CN 1823807 published on the 30.08.2006. The invention relates to a pharmaceutical composition made as a suppository and containing micronized diosmin and hesperidin. The composition of a 0.5-5 g weight suppository, in grams:

| | |
|---|---|
| Diosmin | 0.002-4.8 |
| Hesperidin | 0.0005-1 |
| Suppository base | 0.01-5 |
| Additional agents | 0-3 |

The suppository base contains: cacao oil, semi synthetic glycerides of fatty acids, glycerogelatin, polyethylene glycol, polyoxyethylene monostearate ester, taken alone or as any of their mixtures.

As additional agents, use is made of hardeners, thickeners, absorption accelerators, antioxidants, preservatives, taken alone or as any of their mixtures.

### Summary of the invention

The task to be solved by the present invention consists in widening the raw material base for producing a medicine made as suppositories and containing diosmin and additional substances to treat hemorroids, proctitis or other inflammatory proctology diseases under a medicinal form convenient for usage, vegetal ingredients being used additionally.

The technical result obtained while implementing the present invention consists in the improvement of the therapeutic efficiency of the proctology disease treatment by stopping inflammatory processes in tissues, phenomena of edema, pruritus, burning and the remission period increase. The medicinal agent restores the normal condition of tissues in the ano-rectal area and provides for a high efficiency without any side-effects or complications; the combination of diosmin, the active ingredient, with active natural additives provides for a combined effect on all the organs of the ano-rectal area, and, as a consequence, an improvement of the venous outflow and for a stable medical effect.

Said technical result is achieved thanks to the fact that the medicinal agent made as a suppository to treat hemorrhoid, proctitis and other inflammatory proctologic diseases comprises diosmin, green tea extract and dexpanthenol as active agents, and an emulsifier and fatty acid glycerides as additional substances, at the total mass of a suppository of 1.35 to 3.65 g and at the following ingredient ratio, in grams:

| | |
|---|---|
| Diosmin | 0.3-0.65 |
| Dexpanthenol | 0.05-0.2 |
| Green tea extract | 0.05-0.2 |
| Emulsifier | 0.0135-0.1825 |
| Fatty acid glycerides | Balance. |

Rectal suppositories contain solid fat, witepsol or supposyr as fatty acid glycerides.

In this case, the suppositories obtained have the following physicochemical characteristics:
Color - light-yellow.
Suppository shape: torpedo-like, with a smooth wax-like surface.
Melting point: not higher than 37°C.
Full deformation time: no more than 15 min.

Said preparation relates to the class of substances controlling venule tonicity, improving local trophicity of tissues and reducing inflammatory reactions in tissues.

The mechanism of the medicinal effect of the preparation is linked to the stabilization of the cellular membrane in the blood microcirculation channel, to the improvement of rheology properties of the capillary zone in the ano-rectal area and to the blocking of the inflammatory reaction in the area of hemorrhoid nodes, which finally leads to stoppage of pathological processes in the ano-rectal area, to the normalizing of the tissue metabolism and to a reverse evolution of pathological processes.

Said preparation has a complex therapeutic effect on tissues and provides for antimicrobial, local anesthetic, anti-inflammatory, wound-healing, adaptive, anti-tumor effects.

The additives and the pharmaceutically admissible base for the medicinal form of the present invention were selected experimentally.

It was established that high results related to the compatibility of the active agents with the suppository base and to the medical effect were achieved with the use of fatty acid glycerides taken as a suppository base. According to one embodiment of the invention, the suppositories can contain, as a suppository base, fatty acid glycerides such as solid fat. According to another embodiment, witepsol or supposir were used as a suppository base.

It is necessary to note that the pharmacological properties of individual ingredients of said medicinal preparation used in the present combination give a high therapeutic effect, which is confirmed by preclinical tests of the preparation.

Addition of diosmin to the preparation for treating hemorrhoid, proctitis and other inflammatory pathological diseases favor the increase of the vein tonicity, the decrease of the venous congestion and of the venous stasis volume. Besides, the content of the same with said dosage has a vasoconstrictive effect on the veins and presents a dosage-dependent property; this fact provides for a higher resistance of capillaries, the permeability of the same being decreased and the tissue microcirculation is improved. Diosmin demonstrates an angioprotective effect which leads to a higher vascular resistance, a higher systolic and diastolic pressure in the cases of orthostatic hypertension within the postoperative period and to an improvement of the lymphatic drainage of the vessels.

The use of dexpanthenol as an additive to the preparation stimulates the regeneration of the skin and of the mucous membrane, normalizes the cellular metabolism, accelerates the mitosis and increases the strength of the collagenous fibers, as well as it makes a regenerative, metabolic and slight anti-inflammatory effect.

The addition of a green tea extract (camellin), an active natural antioxidant, to the suppository composition prevents any injury of cellular structures by free radicals, it performs an anti-inflammatory and antioxidant effect. The camellin substance is produced from a dried extract of green tea obtained from Chinese tea (Camellia sinensis) leaves of the Theaceae family; as to its appearance, it is a finely divided powder colored from greenish yellow to dark brown, with a slight specific odor. In its composition, Camellin contains natural polyphenol compounds having their main structure C6-C3-C6, in a ratio of at least 75%. The main substances are derivatives of flavan-3-ol and are represented by epicathechin, catechin gallate, epicathechin gallate, gallocathechin gallate, and derivatives of flavanon-3-ol such as quercetin, dihydroquercetin.

Identification and determining of the quantitative content of the polyphenol amount are based on oxidation-reduction reactions provided with phenolic groups. Antioxidant properties are determined by the presence of a high number of phenolic groups in the structure of green tea catechins that represent "traps" for free radicals (N.K. Zenkov, N.V. Kandalintseva, V.Z. Lankin, E.B. Menshikova, A.E. Prosenko. Phenolic bioantioxidants - Novosibirsk, 2003). As to the pharmacological effect, the polyphenols of the camellin substance are powerful natural antioxidants showing a wide range of biological activity.

The use of an emulsifier in this preparation provides for the possibility to produce a medicinal form of a high quality, it enables one to achieve homogenous distribution of medicinal substances in the human's rectum, it reduces the base fragility and effects an influence on the rate of medicinal substances release while prolonging the effect of these substances on the organism. The quantitative emulsifier index within 0.0135 to 0.1825 g is sufficient and necessary to obtain a high quality of the medicinal form with a mass of 1.35 to 3.65 g.

### Embodiments of the invention

The technology of producing suppositories consists in the following.

The ingredients prepared are loaded into a capacity for preparing the mass for suppositories, where the ingredients are melted and stirred.

The suppository base is a solid fat or witepsol, or supposir, and it is melted together with the emulsifier at the temperature of 60-65°C, the mixture is stirred and cooled to the temperature 42-43°C. The active pharmaceutical substances (APS): diosmin, dexpanthenol, green tea extract (camellin) are loaded into a prepared base and homogenized until getting a homogenous mass.

The suppository mass obtained is packed into a cellular package of a polyvinyl chloride film (EP-73 type). The temperature of the suppository mass is 42-45°C. The average mass of a suppository is 2.0 g.

The suppositories obtained have brown color with admissible color non-homogeneity and have a torpedo-like shape. The melting point is not higher than 37°C.

### Example 1

Composition for 1 suppository with the mass of 1.8 g:

| | |
|---|---|
| Diosmin | 0.3 |
| Dexpanthenol | 0.2 |
| Green tea extract | 0.1 |

Suppository base:

| | |
|---|---|
| Emulsifier | 0.12 |
| Solid fat | (Balance) |

### Example 2

Composition for 1 suppository with the mass of 2.0 g:

| | |
|---|---|
| Diosmin | 0.4 |
| Dexpanthenol | 0.1 |
| Green tea extract | 0.2 |

Suppository base :

| | |
|---|---|
| Emulsifier | 0.1 |
| Witepsol | (Balance) |

### Example 3

Composition for 1 suppository with the mass of 3.5 g :

| | |
|---|---|
| Diosmin | 0.65 |
| Dexpanthenol | 0.05 |
| Green tea extract | 0.2 |

Suppository base :

| | |
|---|---|
| Emulsifier | 0.05 |
| Supposir | (Balance) |

The rectal suppositories passed preclinical probations at "OLFARM", LLC., of a testing laboratory accredited by the Federal Agency for technical regulation and metrology as a technically competent and independent testing laboratory, Accreditation certificate No POCC RU.0001.21FL10 of October 09, 2009.

The license for performing work with microorganisms of the 3-4^{th} pathogenicity groups No 77.01.13.001.L.000142.04.09 of April 30, 2009.

Conclusions of the Report of preclinical tests of the preparation requested:
1. Acute and subchronic toxicity, aftereffect and local irritating effect, allergenic properties and specific activity of the "Gemodan Rectal Suppositories" produced by "Altfarm" LLC (Russia) were investigated and evaluated.
2. The study was carried out on three species of laboratory animals: white rats of no breed of both sexes, with a total number of 196 (98 females + 98 males), rabbits of both sexes and guinea pigs.
3. The study of the acute toxicity of the investigated preparation "Gemodan Rectal Suppositories" on white rats at rectal administration could not provide for determining the LD50. The maximal practicable dosage of 4000 mg/kg is perfectly endured and does not cause any intoxication signs immediately after administering or within 14 following observation days.
4. Subchronic toxicity was determined on rabbits of both sexes. The preparation under investigation was administered rectally once a day for 30 days with 3 dosages: 13 mg/kg (single dosage), 65 mg/kg (quintuple dosage) and 130 mg/kg (tenfold dosage).
5. In the case of rectal administering of the investigated preparation, no lethal cases were recorded for all the 3 dosages. No trustworthy changes were observed in the relative mass of the internal organs: liver, kidneys, adrenal glands, spleen, thymus, stomach, pancreas, heart, lungs, brain. No visible signs of intoxication were reported. The body mass gain or the temperature of animals receiving the preparation with all the 3 dosages did not differ from respective indices of the control animals that received placebos.
6. It was shown that rectal administering of the investigated preparation for 30 days with all the 3 dosages does not cause any statistically significant differences in such biochemical and hematological parameters as the content of total protein, glucose, urea, creatinine, activity of AlT, AsT enzymes and of alkaline phosphatase, hemoglobin concentration, hematocrit, the content of erythrocytes, leukocytes, reticulocytes and thrombocytes, as well as the parameters of the differential blood count.
7. The study of the aftereffect of the investigated preparation carried out two weeks after the end of the 30-days rectal administering showed that all the indices studied on the animals receiving the investigated preparation in the dosages of 13, 65 and 130 mg/kg, did not differ from the respective indices of the placebo group animals after 2 weeks following the end of the administering period.
8. The results of the autopsy showed that the experiments for determining subchronic toxicity did not demonstrate any morphological changes in the liver, kidneys, adrenal glands, spleen, thymus, stomach, pancreas, heart, lungs, ovaries, testis or in brain tissues, due to the effect of the investigated preparation..
9. A comparative histological study showed that no development of cytological changes under the effect of the investigated preparation was observed in studied internal organs or tissues.
10. The study of the local irritating effect carried out on rabbits of both sexes showed that the administration of the rectal suppositories "Gemodan" for 30 days does not have any irritating effect on the mucous membrane of the rectum.
11. The study of allergenic properties of the preparation "Rectal suppositories Gemodan" carried out by means of the evaluation of the cutaneous anaphylaxis and of a conjunctival test on guinea pigs showed that the preparation under study does not show any allergenic effect.
12. The results of analyzing the mutagenic and promutagenic activity by the Ames test (Method of evaluating back mutations in Salmonellatyphimurium bacteria) showed that the specimens of the substances Camellin, Diosmin, Panthenol do not cause back mutations of the indicator strains TA 98, TA 100, TA 102, TA 1535, TA 1537 in dosages 0.125-10 mg per dish. A specimen of the medicinal agent "Gemodan Rectal Suppositories" with dosages of 6-50 mg per dish neither causes any mutagenic effect.
13. The study of the antimicrobial effect showed that the preparation "Rectal suppositories Gemodan", series 010613, manufacturer "ALTFARM" LLC, Russia, and the substance Camellin dry extract, series 010613, manufacturer "ALTFARM" LLC, Russia, demonstrated antimicrobial activity against bacterial cultures and have a low activity against Candida spp. The values of the minimal inhibiting concentration (MIC) of the active ingredients making part of the preparation, such as Diosmin and Camellin, are 100 to 2000 times lower than their content in this medicinal form.
14. In the studies of the local anesthetic activity according to the Renier method on the rabbit eye cornea, an average value of the Renier index of 190±14 for the investigated preparation was obtained. Thus, the presence of a slight local anesthetic activity of the Gemodan preparation was showed. The same experiment established that the preparation under study has no local anesthetic effect on the rabbit eye cornea.
15. The study of the anti-inflammatory activity of the preparation was carried out with the help of a model of an acute exudative inflammation such as a formalin-induced edema of a paw in rats. It was shown that the preparation "Rectal suppositories Gemodan" demonstrates a significant anti-inflammatory activity reducing a paw edema in rats, induced by a formalin injection.

On the basis of the results of evaluating the acute and subchronic toxicity, the aftereffect and the local irritating effect, as well as the allergenic properties, the mutagenic activity and the specific pharmacological activity (antimicrobial, local anesthetic and anti-inflammatory) of the investigated preparation "Rectal suppositories Gemodan", obtained with the use of biochemical, hematological and histological methods of investigation, the considered preparation can be recommended to carry out clinical studies in The Russian Federation.

The suppositories of the present invention can be recommended for treating other inflammatory proctology diseases that are related to the inflammation of rectum tissues and to a venous outflow from said tissues (for example, anal fissures, erosion on the surface of internal hemorrhoids, thrombosis of hemorrhoids, acute periproctitis).

The results of the studies enable one to conclude that the suppositories of the present invention demonstrate anti-inflammatory and regenerating properties. The presentation of the medicinal agent under the form of a suppository provides for a prolonged release of active agents into the cavities of humans and provides its effect for a long time period.

The advantage of the present invention consists in its combined effect onto the ano-rectal region thanks to its active combination of ingredients, in the improvement of the venous outflow, in the achieving a stable healing effect, together with a reduced need to provide enteral and parenteral administration of necessary preparations thanks to its local effect onto the tissues. The suppositories do not cause side effects. The independent administering this medicinal form as a suppository is convenient for treating a patient in ambulatory conditions.

## Claims

1. Medicinal agent made as a rectal suppository to treat hemorrhoid, proctitis and other inflammatory proctology diseases, containing diosmin as an active ingredient and an emulsifier and fatty acid glycerides as auxiliary substances, **characterized in that** it additionally contains, as active ingredients, green tea extract and dexpanthenol, with the total mass of a suppository of 1.35 to 3.65 g and with the following ingredient ratio, in g:
| | |
|---|---|
| diosmin | 0.3-0.65 |
| dexpanthenol | 0.05-0.2 |
| green tea extract | 0.05-0.2 |
| emulsifier | 0.0135-0.1825 |
| Fatty acid glycerides | Balance. |

2. Medicinal agent made as a rectal suppository to treat hemorrhoid, proctitis and other inflammatory proctology diseases of claim 1, **characterized in that** the use is made of a solid fat, witepsol or supposir as fatty acid glycerides.
